(19) Europäisches Patentamt
European Patent Office
Office européen des brevets

(11) Publication number: 0 198 696 B1

(12) # EUROPEAN PATENT SPECIFICATION

(45) Date of publication of patent specification: 11.04.90

(21) Application number: 86302749.6

(22) Date of filing: 14.04.86

(51) Int. Cl.[5]: C 07 F 9/50, C 07 F 9/58, C 07 H 23/00, A 61 K 31/66, A 61 K 31/70

(54) **Phosphine compounds.**

(30) Priority: 16.05.85 US 734524
14.02.86 US 829359
16.04.85 US 723778
20.05.85 US 736018

(43) Date of publication of application:
22.10.86 Bulletin 86/43

(45) Publication of the grant of the patent:
11.04.90 Bulletin 90/15

(84) Designated Contracting States:
AT BE CH DE FR GB IT LI LU NL SE

(56) References cited:
EP-A-0 151 046
EP-A-0 164 970
JOURNAL OF MEDICINAL CHEMISTRY, vol. 9, no. 3, May 1966, pages 414-416; R.F: STRUCK et al.: "Tertiary phosphines and phosphine oxides containing a 2-haloethyl group"

The file contains technical information submitted after the application was filed and not included in this specification

(73) Proprietor: SMITHKLINE BEECHAM CORPORATION
P.O. Box 7929 1 Franklin Plaza
Philadelphia Pennsylvania 19101 (US)

(72) Inventor: Hill, David Taylor
109 Magnolia Place
North Wales Pennsylvania 19454 (US)
Inventor: Johnson, Randall Keith
71 Llanfair Circle
Ardmore Pennsylvania 19003 (US)

(74) Representative: Waters, David Martin, Dr. et al
Smith Kline & French Laboratories Ltd. Patent Department Mundells
Welwyn Garden City Hertfordshire AL7 1EY (GB)

(56) References cited:
THE AMERICAN JOURNAL OF MEDICINE, vol. 75, 30th December 1983, pages 109-113; S.T. CROOKE et al.: "Molecular mechanisms of action of auranofin and other gold complexes as related to their biologic activities"

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 198 696 B1

Courier Press, Leamington Spa, England.

## Description

### Background of the Invention

This invention relates to novel phosphine compounds which have tumor cell growth-inhibiting activity, pharmaceutical compositions containing an effective, tumor cell growth-inhibiting amount of such a phosphine compound, and their use for treating tumor cvells sensitive to such a compound by administering tumor cell growth-inhibiting amounts of a phosphine compound to a host animal afflicted by such tumor cells.

The heterocyclic phosphine compounds of this invention are not known. Cariati et al., *Inorg. Chim. Acta, 1* (2), 315—18 (1967), Bates et al., *Inorg. Chim. Acta, 81* (2), 151—156 (1984), and McAuliffe et al., *J. C. S. Dalton,* 1730 (1979), disclose bis[1,2-bis(diphenylphosphino)ethane]gold(I) chloride.

The digold compounds comprised by some of the pharmaceutical compositions of this invention are known. Sadler et al., *J. Chem. Soc., Dalton Trans,* 969—974 (1984), propose the synthesis of dichlorobis[1,2-bis(diphenylphosphino)ethane]digold(I), but were unable to isolate the compound. Schmidbaur et al., *Chem. Ber, 110,* 2751—2557 (1977), disclose dichlorobis[1,2-bis(diphenylphosphino)methane]digold(I). Stringer et al., abstract from the 15th Middle Atlantic Regional Meeting of the American Chemical Society, January 7—9, 1981, Washington, D.C., disclose the synthesis of dichlorobis[1,2-bis(diphenylphosphino)-ethane]digold(I), dichlorobis[1,2-bis(diethylphosphino)ethane]digold(I), and diperchloratobis[1,2-bis(di-ethylphosphino)ethane]digold(I), and state that these three compounds were evaluated as oral pharmaceutical compositions for activity in adjuvant-induced arthritis in the Charles River wistar rat. However, there is no disclosure in the Stringer et al. reference that such compounds actually have antiarthritic or any other therapeutically useful biological activity.

Struck et al., *J. Med. Chem., 9,* 414—416 (1966), disclose cytotoxic activity for 1,2-bis(diphenyl-phosphino)ethane which is used as a starting material for preparing some of the pharmaceutical compositions and methods of treatment of the subject invention.

None of the aforementioned references disclose or suggest the compounds, pharmaceutical compositions or therapeutic use of the instant invention.

### Summary of the Invention

This invention relates to compounds of the formula:

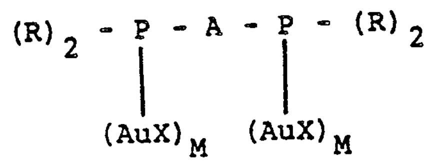

FORMULA (I)

wherein:

R is the same and is 2-pyridyl, 4-pyridyl, 2-thienyl or 2-furyl;

X is the same and is halo or thiosugar;

M is 0 or 1, provided that when M is 1, R is 2-pyridyl or 4-pyridyl; and

A is a straight or branched alkanediyl chain of from one to six carbon atoms.

When X is thiosugar, the attachment of X to the gold atom is through the sulfur atom of the thiosugar.

This invention also relates to a compound of the formula

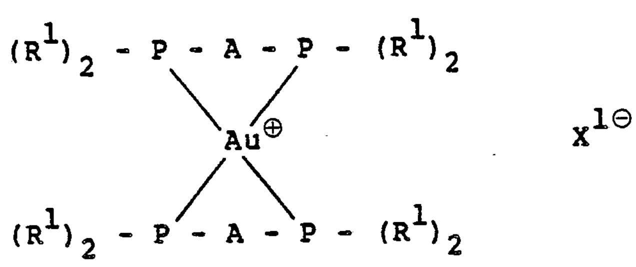

FORMULA (II)

wherein:

$R^1$ is the same and is 2-pyridyl;

A is the same and is a straight or branched alkanedoyl chain from one to six carbon atoms; and

$X^1$ is the same and is halo.

This invention also relates to a pharmaceutical composition which comprises an effective, tumor cell growth-inhibiting amount of an active ingredient and an inert, pharmaceutically acceptable carrier or diluent, wherein said composition is useful for inhibiting the growth of animal tumor cells sensitive to the active ingredient, and wherein the active ingredient is a compound of Formula (I) or a compound of the formula:

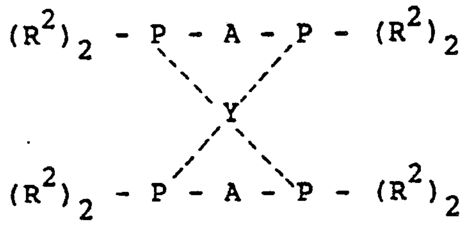

FORMULA (III)

wherein:

$R^2$ is the same and is phenyl, ethyl or 2-pyridyl;

A is a straight or branched alkanediyl chain of from one to six carbon atoms;

Y is a link between the four P atoms of structure

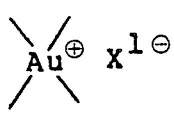 or 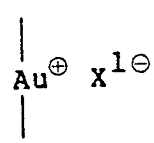 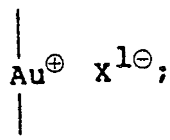

and

$X^1$ is halo;

provided that when Y is 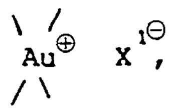

$R^2$ is 2-pyridyl;

and a pharmaceutically acceptable carrier.

Another aspect of this invention relates to the use in inhibiting the growth of animal tumor cells of a compound of Formula (I) or Formula (III) which use comprises administering to an animal afflicted with said tumor cells, an effective, tumor cell growth-inhibiting amount of a compound of Formula (I) or Formula (III).

Detailed Description of the Invention

It will be appreciated by one of skill in the art that all the compounds of Formula (II) are encompassed by the scope of Formula (III).

By the term "thiosugar" is meant any 1-thioaldose. Examples of such thiosugars include 1-thioglucose, 1-thiogalactose, 1-thiomannose, 1-thioribose, 1-thiomaltose, 1-thiofucose, tetra-*O*-acetyl-1-thioglucose, tetra-*O*-acetyl-1-thiomannose, tetra-*O*-acetyl-1-thiogalactose, tri-*O*-acetyl-1-thioribose, hepta-*O*-acetyl-1-thiomaltose, and tri-*O*-acetyl-1-thiofucose.

All the compounds of Formulas (I), (II) and (III) can be prepared by methods available to one skilled in the art.

Generally, the compounds of Formula (I) wherein M is 0 can be prepared by reacting the appropriate heterocycle in an anhydrous ethyl ether with an organolithium reagent, such as n-butyl lithium in a non-reactive organic solvent, with the appropriate compound of the formula:

$$Cl_2—P—A—P—Cl_2$$

Formula (A)

wherein A is as defined above.

All the necessary heterocycles, organolithium reagents and Formula (A) compounds are available from commercial sources, for example, from Strem Chemicals, Inc., Newburyport, Massachusetts.

Generally, the starting materials for the Formula (I) compounds wherein M is 1 and X is chloro are the corresponding diphosphino hydrocarbons of Formula (I) wherein M is 0. To obtain the digold products of Formula (I), an appropriate diphosphino hydrocarbon of Formula (I) is reacted either directly with chloroauric acid tetrahydrate or a reduced form of the acid hydrate obtained by treatment with thiodiglycol in an appropriate non-reactive organic solvent.

To obtain the Formula (I) compounds wherein X is thiosugar, the appropriate Formula (I) compound wherein X is chloro is reacted with the appropriate sodium thiosugar. The necessary sodium thiosugars are commercially available, for example, from Sigma Chemical Co., St. Louis, Missouri, or are made by conventional techniques.

Formula (I) compounds wherein X is bromo are prepared by reacting the appropriate ligand of Formula (I) wherein M is 0 with bromoauric acid hydrate, (which is commercially available, for example, from Strem Chemicals, Inc., Newburyport, Massachusetts), which has been reduced by treatment with thiodicylcol; or by reacting the appropriate ligand of Formula (I) wherein M is 0 with bromoauric acid hydrate directly in an appropriate non-reactive organic solvent. Alternatively, Formula (I) compounds wherein X is bromo are prepared by reacting the appropriate compound of Formula (I), wherein X is chloro, with sodium bromide in an appropriate organic solvent, such as aqueous ethanol or DMF.

Formula (I) compounds wherein X is iodo are prepared by treating the appropriate compound of Formula (I), wherein X is chloro or bromo, with sodium iodide in an appropriate organic solvent such as acetone.

Generally, the Formula (II) compounds can be obtained by reacting the appropriate Formula (I) compound wherein M is 0 or 1 with the appropriate Formula (I) compound wherein M is 0 in an appropriate non-reactive organic solvent.

Generally, the compounds of Formula (III) wherein Y is

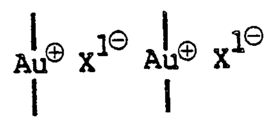

and $X^1$ is chloro, can be prepared by reacting one mole of the appropriate ligand of the formula:

$$(R^3)_2—P—A—P—(R^3)_2$$

Formula (B)

with one mole of the appropriate gold complex of the formula:

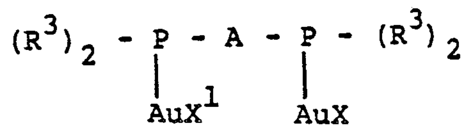

Formula (C)

wherein $R^3$ is the same and is phenyl or ethyl; $X^1$ is halo and A are defined as above, in a non-reactive organic solvent.

All the necessary Formula (B) ligands are available from commercial sources, for example, from Strem Chemicals, Inc., Newburyport, Massachusetts.

The Formula (C) gold complexes, wherein $X^1$ is chloro, are conveniently prepared by reacting the appropriate ligand of Formula (B) with chloroauric acid tetrahydrate which has been reduced by treatment with thiodiglycol. Formula (C) gold complexes, wherein $X^1$ is chloro, may also be conveniently prepared by reacting the appropriate ligand of Formula (B) directly with chloroauric acid hydrate in an appropriate non-reactive organic solvent.

Formula (C) complexes, wherein $X^1$ is bromo, are prepared by reacting the appropriate ligand of Formula (B) with bromoauric acid hydrate (which is commercially available, for example, from Strem Chemicals, Inc., Newburyport, Massachusetts) which has been reduced by treatment with thioidiglycol, or by reacting the appropriate ligand of Formula (B) directly with bromoauric acid hydrate in an appropriate non-reactive organic solvent. Alternatively, Formula (C) complexes, wherein $X^1$ is bromo, are prepared by reacting the appropriate compound of Formula (C), wherein $X^1$ is chloro, with sodium bromide in an appropriate organic solvent, such as aqueous ethanol or DMF.

Formula (III) compounds, wherein $X^1$ is bromo, are prepared by reacting one mole of the appropriate compound of Formula (C), wherein $X^1$ is bromo, with one mole of the appropriate compound of Formula (B). Alternatively, Formula (III) compounds, wherein $X^1$ is bromo, are prepared by reacting the appropriate Formula (III) compound, wherein $X^1$ is chloro, with sodium bromide in an appropriate organic solvent, such as aqueous ethanol or DMF.

Formula (III) compounds wherein $X^1$ is iodo, are prepared by reacting the appropriate Formula (III) compound, wherein $X^1$ is chloro or bromo, with sodium iodide in an appropriate organic solvent, such as acetone.

The compounds of Formula (I) and (III) have tumor cell growth-inhibiting activity which has been demonstrated in at least one animal tumor model.

P388 lymphocytic leukemia is currently the most widely used animal tumor model for screening for anti-tumor agents and for detailed evaluation of active compounds. This tumor system is widely accepted as an antitumor agent screening tool because it is sensitive to virtually all of the clinically active antineoplastic agents; quantitative and reproducible; amenable for large-scale screening; and predictive for activity in other animal tumor models. Drugs that are highly active in intraperitoneal (ip) P388 leukemia are generally active in other tumor models as well. The anti-tumor activity of the compounds of Formula (I) and (III) is demonstrated in the P388 leukemia mouse model employing the following protocol:

$10^6$ P388 leukemia cells are inoculated ip in $B6D2F_1$ mice. Twenty-four hours later, if the tumor inoculum proves to be free of bacterial contamination (as determined by 24 hours incubation in thioglycollate broth), animals are randomized into groups of 6 and housed in shoebox cages. The compound to be evaluated is dissolved in saline if soluble therein or in a minimal volume of either N,N-dimethyl-acetamide (DMA) or 95% ethanol (depending upon solubility). An equal volume of saline is added to those compounds dissolved in an organic vehicle; if the drug comes out of solution an equal volume of

polyethoxylated castor oil is added and then saline qs to a concentration such that the desired dose is delivered in 0.5 ml. The final concentration of DMA, ethanol and/or polyethoxylated castor oil is ≥ 10 percent. Dilutions for lower doses are made with saline so there is a decreasing proportion of organic solvents in the vehicle with decreasing dosage. These vehicles provide soluble formulations (or suspensions). Formulations are prepared immediately prior to injection. The compound is administered ip on Days 1 through 5 (i.e. treatment is initiated 24 hrs after tumor inoculation). Each experiment includes three groups of 6 animals as untreated controls and animals treated with a positive control, cisplatin, at two dose levels. Animals are weighed as a group on Days 1, 5 and 9, and average weight change (Δ wt.) is used as a reflection of toxicity. Each experiment also includes an inoculum titration — groups of 8 mice inoculated ip with $10^5$ to $10^0$ P388 leukemia cells. The titration is used to calculate cell kill achieved by treatment with drugs. Animals are monitored daily for mortality and experiments are terminated after 45 days. The endpoint is median survival time (MST) and increase in lifespan (ILS) which is the percentage of increase in MST relative to untreated controls. Untreated controls inoculated ip with $10^6$ P388 leukemia cells generally survive for a median of 9 to 11 days. A drug is considered active if it produces ≥ 25 percent ILS.

A summary of the evaluation of several compounds of Formula (I) in the *in vivo* ip P388 model is shown in the following Table A.

TABLE A

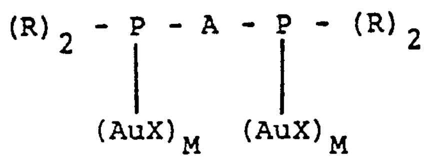

FORMULA (I)

| Compound No. | R | A | M | X | MTD[a] (mg/kg) | ILS (max)[b] % |
|---|---|---|---|---|---|---|
| 1 | 2-pyridyl | $(CH_2)_2$ | 0 | — | 16 | 30/20 |
| 2 | 4-pyridyl | $(CH_2)_2$ | 0 | — | 32 | 35/37 |
| 3 | 2-thienyl | $(CH_2)_2$ | 0 | — | 16 | 60/55 |
| 4 | 2-furyl | $(CH_2)_2$ | 0 | — | 64 | 60/30/50 |
| 5 | 2-pyridyl | $(CH_2)_2$ | 1 | Cl | 6 | 55/60 |
| 6 | 4-pyridyl | $(CH_2)_2$ | 1 | Cl | 12 | 65/32/28 |
| 7 | 2-pyridyl | $(CH_2)_2$ | 1 | 1-thio-glucose | 8 | 85/53/56 |

[a]maximally tolerated dose for B6D2F female mice on an ip qDx5 regimen.
[b]maximum increase in lifespan produced in mice bearing ip P388 leukemia (figures separated by slashes indicate data generated in separate experiments).

Based on the data set forth in Table A, compounds of Formula (I) showed significant anti-tumor activity in the *in vivo* ip P388 leukemia tumor assay.

A summary of the evaluation of several compounds of Formula (III) in the *in vivo* ip P388 model is shown in the following Table B.

TABLE B

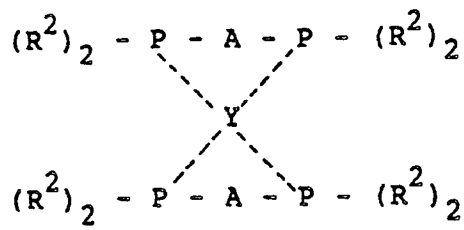

FORMULA (III)

| Compound No. | $R^2$ | A | Y | | $X^1$ | MTD[(a)] (mg/kg) | ILS (max)[(b)] (%) |
|---|---|---|---|---|---|---|---|
| 8 | phenyl | $(CH_2)_2$ | $Au^+X^{1-}$ | $Au^+X^{1-}$ | Cl | 8 | 81/91/55 |
| 9 | ethyl | $(CH_2)_2$ | $Au^+X^{1-}$ | $Au^+X^{1-}$ | Cl | 16 | 29 |
| 10 | 2-pyridyl | $(CH_2)_2$ | $Au^+X^{1-}$ | | Cl | 8 | 80/75/83/84 |

[(a)]maximally tolerated dose for B62DF female mice on an ip pDX5 regimen
[(b)]maximum increase in lifespan produced in mice bearing ip P388 leukemia (figures separated by slashes indicate data generated in separate experiments).

Based on the data set forth in Table B, compounds of Formula (III) showed significant anti-tumor activity in the *in vivo* ip P388 leukemia tumor assay. It should be noted that the 4-pyridyl analog of Compound No. 10 of Table B was also tested twice in the ip P388 leukemia assay, but exhibited insignificant anti-tumor activity (i.e. $<25\%$) in both tests.

The cytotoxic activity of two Formula (III) compounds was evaluated *in vivo* using B16 melanoma cells. In this system, groups of eight $B6D2F_1$ mice are inoculated ip with 0.5 ml of a 10% (w:v) brei of B16 melanoma prepared from pooled sc tumors excised at 14—21 days from C57B1/6 donor mice. Daily treatment is begun 24 hours after tumor implantation and is continued daily for ten (10) days. The route of drug administration is ip. The mice are monitored daily for survival for sixty (60) days. Anti-tumor activity is assessed by prolongation of median survival time. An ILS of $\geq 25\%$ indicates activity in this tumor model.

A summary of the evaluation of two compounds of Formula (III) in the *in vivo* ip B16 melanoma assay is shown in Table C.

TABLE C

| Compound No.[(a)] | MTD (mg/kg) | ILS (%)[(c)] |
|---|---|---|
| 8 | 4[(b)] | 35 |
| 10 | 10[(d)] | 43 |

[(a)]see Table B for structure.
[(b)]maximally tolerated dose for $B6D2F_1$ mice on ip qDx10 regimen.
[(c)]maximum increase in lifespan produced in mice bearing ip B16 melanoma.
[(d)]maximally tolerated dose for $B6D2F_1$ mice on ip qDx9 regimen (supply of compound No. 10 was depleted on day 9).

The pharmaceutical composition of this invention comprises an effective, tumor cell growth-inhibiting amount of a compound of Formula (I) or Formula (III) and an inert pharmaceutically acceptable carrier or diluent. These compositions are prepared in dosage unit form appropriate for parenteral administration.

Compositions according to the invention for parenteral administration include sterile aqueous or nonaqueous solutions, suspensions or emulsions. The composition may be in the form of a solution of the

active ingredient in a minimal volume of dimethylacetamide or ethanol, for example, 5% v/v, brought up to volume with peanut oil or normal saline solution. Polyethoxylated castor oil, for example, 2 to 5% v/v, may also be used to solubilize the active ingredient. In addition, the composition may be in the form of a slurry with, for example, hydroxypropyl cellulose or other suitable suspending agent. As an emulsifying agent, lecithin, for example, may be used. The composition may also be provided in the form of a sterile solid which can be dissolved in a sterile injectable medium immediately before use.

Friereich et al., *Cancer Chemo. Rept., 50,* 219—244 (1966), compared the quantitative toxicity of 18 anticancer drugs in six species after correcting the data to a uniform schedule of treatment for five consecutive days. This analysis demonstrated that mouse, rat, dog, human, monkey and man have essentially the same maximum tolerated dose (MTD) when compared on a basis of $mg/m^2$ of body surface area. The study suggested that Phase I clinical trials could be safely initiated at a dose one-third the animal MTD. The mouse was as useful as any other species in this regard on which to base the calculation. The appropriate therapeutically effective dose for any compound of the invention can therefore be determined readily by those skilled in the art from simple experimentation with laboratory animals, preferably mice.

It will be appreciated that the actual preferred dosages of the compounds of Formula (I) or Formula (III) used in the compositions of this invention will vary according to the particular compound being used, the particular composition formulated, the mode of administration, and the particular site, host and disease being treated. The route of internal administration should be selected to ensure that an effective tumor cell growth-inhibiting amount of the compound of Formula (I) or Formula (III) contacts the tumor. Optimal dosages for a given set of conditions can be ascertained by those skilled in the art using conventional dosage determination tests in view of the above experimental data. For parenteral administration of a compound of Formula (III) or a gold-containing compound of Formula (I) the dosage generally employed is from about 5 to about 100 $mg/m^2$ of body surface per day for one to five days, repeated about every fourth week for four courses of treatment. For parenteral administration of a non-gold containing compound of Formula (I) the dose preferably employed is from about 15 to about 600 $mg/m^2$ of body surface per day for five days, repeated about every fourth week for four courses of treatment.

## Examples

The following examples illustrate the chemical preparation of several compounds of Formula (I) and Formula (III) which are used in the compositions and methods of this invention and as such are not to be construed as limiting the scope thereof. All temperatures are in degrees Centigrade.

### Example 1

#### 1,2-Bis(di-2-pyridylphosphino)ethane

Under an argon atmosphere, 2-bromopyridine (30.9 g, 0.19 mole) in anhydrous ethyl ether (50 ml) was added to n-butyl lithium (0.19 mole) in hexane (73 ml) keeping the temperature below −50°. After stirring for 1 hour, an additional 7.5 g of 2-bromopyridine was added, and the mixture was stirred for 30 minutes. An ether solution (100 ml) of 1,2-bis(dichlorophosphino)ethane (10 g, 43 mmole), obtained from Strem Chemicals, Inc., Newburyport, Massachusetts, was added, and the mixture was stirred for 1 hour, at −50°, and then allowed to warm to room temperature overnight. Saturated aqueous ammonium chloride was collected and dissolved in chloroform, dried ($Na_2SO_4$), filtered and the solvent was removed to give a dark residue. The residue was treated with acetone, and then the acetone was cooled to give a light yellow solid (4.5 g). Recrystallization from acetone gave 2.64 g of the named product, melting point (m.p.) 134—135°.

### Example 2

#### 1,2-Bis(di-4-pyridylphosphino)ethane

Under an argon atmosphere, 4-bromopyridine (30.9 g, 0.19 mole) in anhydrous ethyl ether (50 ml) was added to n-butyl lithium (0.19 mole) in hexane (73 ml) keeping the temperature below −50°. After stirring for 1 hour, an additional 7.5 g of 4-bromopyridine was added, and the mixture was stirred for 30 minutes. An ether solution (100 ml) of 1,2-bis(dichlorophosphino)ethane (10 g, 43 mmole), obtained from Strem Chemicals, Inc., Newburyport, Massachusetts, was added, and the mixture was warmed to room temperature overnight. After 18 hours at ambient temperature, aqueous saturated ammonium chloride was added and the solid was removed. The residual solid in the flask was collected, dissolved in chloroform, treated with activated carbon, filtered and the solvent removed. Flash chromatography ($SiO_2$, 7% methanol/methylene chloride) of the residue gave a small amount of the desired product, m.p. 183—185°C.

### Example 3

#### 1,2-Bis(di-2-thienylphosphino)ethane

Under argon at ambient temperature, thiophene (16 g, 0.19 mole) in anhydrous ethyl ether (50 ml) was added to a hexane (73 ml) solution of n-butyl lithium (0.19 moles) which had been diluted with ether (50 ml). After stirring for 1 hour, the mixture was cooled to 0°. Then 1,2-bis(dichlorophosphino)ethane (10 g, 0.043 mole), obtained from Strem Chemicals, Inc., Newburyport, Massachusetts, in ether (50 ml) was added, and the mixture was allowed to warm to room temperature and was stirred for 2 hours. Saturated aqueous ammonium chloride solution was added and the solid material collected. This was dissolved in chloroform, washed with water, dried ($MgSO_4$), filtered and the solvent concentrated to give a solid which

was collected and dried to give 4.2 g, m.p. 108—110°. Chromatography ($SiO_2$, 2:1 $CCl_4/CH_2Cl_2$) gave the named product in analytical purity after crystallization from ethanol; m.p. 114—115°.

Example 4
1,2-Bis(di-2-furylphosphino)ethane

Furan (12.9 g, 0.19 mole) in anhydrous ethyl ether (50 ml) was added to a hexane (73 ml) solution of n-butyl lithium (0.19 moles), diluted with ether (50 ml) and kept at ambient temperature. The mixture was stirred for 2 hours, cooled to 0°, and 1,2-bis-dichlorophosphino)ethane (10 g, 0.043 mole), obtained from Strem Chemicals, Inc., Newburyport, Massachusetts, in ether (50 ml) was added. The mixture was stirred for 18 hours and warmed to room temperature. Saturated aqueous ammonium chloride was added, and the mixture was stirred for 1 hour. The solid was removed. The mixture was extracted with ether, and then chloroform and the extracts were combined. The solid from the combined organic extract was removed and the solvent removed *in vacuo*. The residue was treated with hot hexane, cooled and the solid collected (4.7 g). Re-crystallization from ethanol gave 1.5 g of the named product as needles, m.p. 94—96°.

Example 5

Using the procedure of Example 1 to react the appropriate heterocycle with the appropriate Formula (A) compound, the following compounds of Formula (I) are prepared:

a. 1,2-Bis(di-2-pyridylphosphino)methane
b. 1,2-Bis(di-2-pyridylphosphino)propane
c. 1,2-Bis(di-2-pyridylphosphino)butane
d. 1,2-Bis(di-2-pyridylphosphino)pentane
e. 1,2-Bis(di-2-pyridylphosphino)hexane
f. 1,2-Bis(di-4-pyridylphosphino)methane
g. 1,2-Bis(di-4-pyridylphosphino)propane
h. 1,2-Bis(di-4-pyridylphosphino)butane
i. 1,2-Bis(di-4-pyridylphosphino)pentane
j. 1,2-Bis(di-4-pyridylphosphino)hexane
k. 1,2-Bis(di-2-thienylphosphino)methane
l. 1,2-Bis(di-2-thienylphosphino)propane
m. 1,2-Bis(di-2-thienylphosphino)butane
n. 1,2-Bis(di-2-thienylphosphino)pentane
o. 1,2-Bis(di-2-thienylphosphino)hexane
p. 1,2-Bis(di-2-furylphosphino)methane
q. 1,2-Bis(di-2-furylphosphino)propane
r. 1,2-Bis(di-2-furylphosphino)butane
s. 1,2-Bis(di-2-furylphosphino)pentane
t. 1,2-Bis(di-2-furylphosphino)hexane.

Example 6

As a specific embodiment of a composition of this invention, an active ingredient, such as one part of the compound of Example 1, is dissolved in 5 parts of dimethylacetamide and 5 parts of polyethoxylated castor oil and then normal saline solution qs, and is administered parenterally in one dose of 30 $mg/m^2$ to a host animal afflicted with tumor cells sensitive to that compound.

Example 7
Dichlorobis[1,2-bis(diphenylphosphino)ethane]digold

a) μ-[1,2-bis(diphenylphosphino)ethane]bis[chlorogold(I)]

Chloroauric acid hydrate (1.6 g, 3.8 mmol) in ethanol (20 ml) was added to bis(1,2-diphenylphosphino)ethane (1.83 g, 4,5 mmol), obtained from Strem Chemicals, Inc., Newburyport, Massachusetts, in 1:1 chloroform/ethanol (40 ml) maintained at room temperature. After one hour, the white precipitate was collected, dissolved in methylene chloride, filtered and ethanol added to induce precipitation. After standing, the product was collected and dried to give 0.97 g (50%) of the named gold complex which had a melting point (m.p.) of 291—293°.

b) Dichlorobis[1,2-bis(diphenylphosphino)ethane]digold

A solution of 1,2-bis(diphenylphosphino)ethane (2.31 g, 5.8 mmoles), obtained from Strem Chemicals, Inc., Newburyport, Massachusetts, in chloroform (100 ml) was added to a slurry of μ-[1,2-bis(diphenyl-phosphino)ethane]bis chlorogold) (5.0 g, 5.8 mmoles), prepared as described above, in chloroform (500 ml) maintained at ambient temperature. After 45 minutes, the reaction mixture became homogeneous (clear), and solvent was removed *in vacuo*. The residue was dissolved in a minimum amount of chloroform and ether was added. After cooling, the precipitate was collected and dried to give 3.0 g (41%) of the named product, m.p. 298—302°.

Example 8

Dichlorobis[1,2-bis(diethylphosphino)ethane]digold

a) μ-[1,2-Bis(diethylphosphino)ethane]bis[chlorogold(I)]

Chloroauric acid tetrahydrate (7.88 g) in water (35 ml) was reduced by thiodiglycol (5.5 g) in ethanol (12 ml) at 0°C in standard fashion. A solution of 1,2-bis(diethylphosphino)ethane (2.06 g), obtained from Strem Chemicals, Inc., Newburyport, Massachusetts, in ethanol (15 ml) was added. After stirring for 1 hour, the reaction mixture was poured into 400 ml of ice water and extracted with methylene chloride. The combined extracts were dried ($MgSO_4$), filtered and cooled overnight. The resulting solid was collected and air dried to give 3.0 g of the named product, m.p. 168—170°C.

b) Dichlorobis[1,2-bis(diethylphosphino)ethane]digold

Addition of 1,2-bis(diethylphosphino)ethane (0.31 g, 1.49 mmole), obtained from Strem Chemicals, Inc., Newburyport, Massachusetts, in a single portion to a solution of μ-[1,2-bis(diethylphosphino)ethane]-bis[chlorogold) (1.0 g, 1.49 mmoles), prepared as described above, in chloroform (200 ml) was carried out and the resultant clear solution was stirred for 30 minutes. The solvent was removed *in vacuo*, and the residue recrystallized from chloroform/ether to give 1.0 g (77%) of the named product, m.p. 211—214°.

Example 9

By utilizing the procedure of Example 7(b) or Example 8(b) to react the appropriate gold complex of Formula (C) (prepared according to the procedure of Example 7(a) or Example 7(a) by utilizing the appropriate haloauric acid hydrate) with the appropriate ligand of Formula (B), the following compounds of Formula (III) wherein $X^1$ is chloro or bromo are prepared; and/or by reacting the appropriate Formula (III) compound, wherein $X^1$ is chloro, with sodium bromide in an appropriate organic solvent, such as aqueous ethanol or DMF, the following Formula (III) compounds wherein $X^1$ is bromo are prepared; and by reacting the appropriate Formula (III) compound wherein $X^1$ is chloro or bromo with sodium iodide in an appropriate organic solvent, such as acetone, the following compounds of Formula (III) wherein $X^1$ is iodo are prepared:

a) dichlorobis[1,2-bis(diphenylphosphino)methane]digold
b) dichlorobis[1,2-bis(diphenylphosphino)propane]digold
c) dichlorobis[1,2-bis(diphenylphosphino)butane]digold
d) dichlorobis[1,2-bis(diphenylphosphino)pendant]digold
e) dichlorobis[1,2-bis(diphenylphosphino)hexane]digold
f) dichlorobis[1,2-bis(diethylphosphino)methane]digold
g) dichlorobis[1,2-bis(diethylphosphino)propane]digold
h) dichlorobis[1,2-bis(diethylphosphino)butane]digold
i) dichlorobis[1,2-bis(diethylphosphino)pentane]digold
j) dichlorobis[1,2-bis(diethylphosphino)hexane]digold
k) diiodobis[1,2-bis(diphenylphosphino)ethane]digold
l) dibromobis[1,2-bis(diphenylphosphino)ethane]digold.

Example 10

As a specific embodiment of a composition of this invention, an active ingredient, such as one part of the compound of Example 7, is dissolved in 5 parts of dimethylacetamide and 5 parts of polyethoxylated castor oil and then normal saline solution qs, and is administered parenterally in one dose of 5 mg/m$^2$ to a host animal afflicted with tumor cells sensitive to that compound.

Example 11

μ-[1,2-Bis(di-2-pyridylphosphino)ethane]bis(chlorogold)

Under an argon atmosphere, 2-bromopyridine (30.95 g, 0.19 mole) in anhydrous ethyl ether (50 ml) was added to n-butyl lithium (0.19 mole) in hexane (73 ml) keeping the temperature below −50°. After stirring for 1 hour, an additional 7.5 g of 2-bromopyridine was added, and the mixture was stirred for 30 minutes. An ether solution (100 ml) of 1,2-bis(dichlorophosphino)ethane (10 g, 43 mmole), obtained from Strem Chemicals, Inc., Newburyport, Massachusetts, was added, and the mixture was stirred for 1 hour at −50°, and then allowed to warm to room temperature overnight. Saturated aqueous ammonium chloride was added, and the mixture was stirred for 1 hour. The solid was collected and dissolved in chloroform, dried ($Na_2SO_4$), filtered and the solvent was removed to give a dark residue. The residue was treated with acetone, and the acetone was cooled to give a light yellow solid (4.5 g). Recrystallization from acetone gave 2.64 g of 1,2-bis(di-2-pyridylphosphino)-ethane, melting point (m.p.) 134—135°.

Thiodiglycol (1 g, 8.18 mmole) in water (10 ml)/methanol (30 ml) was added to chloroauric acid tetrahydrate (0.88 g. 2.14 mmole) in water (10 ml) kept at 0°. After stirring for 15 minutes, 1,2-bis(di-2-pyridylphosphino)ethane, (0.43 g, 1.07 mmole), prepared as described above, in acetone (50 ml)/chloroform (10 ml) was added, and the mixture was stirred for 2 hours. Methanol was added, and the product was collected and slurried with $CH_2Cl_2/CHCl_3$, diluted with methanol and cooled. The resulting solid was collected and dried to give 0.38 g (41%) of the named product, m.p. 292—293°.

Example 12

μ-[1,2-Bis(di-2-pyridylphosphino)ethane]bis[(1-thio-β-D-glucopyranosato-S)gold

A mixture of sodium thioglucose (0.33 g, 1.5 mmole) obtained from Sigma Chemical Company, St. Louis, Missouri, and μ-[1,2-bis(di-2-pyridylphosphino)ethane]bis(chlorogold) (0.6 g, 0.69 mole), prepared as described in Example 1, in chloroform (75 ml)/methanol (75 ml)/water (10 ml) was stirred at ambient temperature for 2 hours and the solvent evaporated. The residue was dissolved in chloroform and the precipitate was collected. The solid was dissolved in methanol, filtered and the solvent evaporated. The residue was dissolved in acetone, cooled, and the precipitate was collected and dried to give 0.49 g (60%) of the named compound as a white, amorphous solid.

Example 13

μ-[1,2-Bis(di-4-pyridylphosphino)ethane]bis(chlorogold)

Under an argon atmosphere, 4-bromopyridine (30.95 g, 0.19 mole) in anhydrous ethyl ether (50 ml) was added to n-butyl lithium (0.19 mole) in hexane (73 ml) keeping the temperature below −50°. After stirring for 1 hour, an additional 7.5 g of 4-bromopyridine was added, and the mixture was stirred for 30 minutes. An ether solution (100 ml) of 1,2-bis(dichlorophosphino)ethane (10 g, 43 mmole), obtained from Strem Chemicals, Inc., Newburyport, Massachusetts, was added and the mixture was stirred for 1 hour at −50°, and then allowed to warm to room temperature overnight. After 18 hours at ambient temperature, aqueous saturated ammonium chloride was added and the solid removed. The residual solid in the flask was collected, dissolved in chloroform, treated with activated carbon, filtered, and then the solvent was removed. Flash chromatography ($SiO_2$, 7% methanol/methylene chloride) of the residue gave a small amount of 1-2-bis(di-4-pyridylphosphino)ethane, m.p. 183—185°.

Thiodiglycol (2 g, 16.4 mmole) in water (10 ml)/methanol (30 ml) was added to chloroauric acid tetrahydrate (1.58 g, 3.83 mmole) in water (10 ml) kept at 0°. After stirring for 15 minutes, 1-2-bis(di-4-pyridylphosphino)ethane (0.77 g, 1.9 mmole), prepared as described above, in $CH_2Cl_2$ (10 ml)/methanol (30 ml) was added and the mixture allowed to stir for 1 hour. Methanol was added, and the precipitate collected and stirred in acetonitrile, filtered and dried to give 0.75 g (45%) of the named product, m.p. 241—242°.

Example 14

Chlorobis[1,2-bis(di-2-pyridylphosphino)ethane]gold

A solution of 1,2-bis(di-2-pyridylphosphino)ethane (0.17 g, 0.42 mmole), prepared as described in Example 1, in $CH_2Cl_2$ (25 ml) was added to a suspension of μ-[1,2-bis(di-2-pyridylphosphino)ethane]bis-(chlorogold) (0.12 g, 0.14 mmole) in $CH_2Cl_2$ (25 ml), and the mixture was stirred at ambient temperature for 18 hours giving a clear solution. The solvent was evaporated, and the residue was dissolved in methanol and diluted with ethyl ether. After cooling, the precipitate was collected and dried to give 0.28 g (98%) of the named product, m.p. 257—258°.

Example 15

a) Using the procedure outlined in Example 11 or Example 13, by directly reacting the appropriate haloauric acid hydrate with the appropriate diphosphino hydrocarbon compound of Formula (I) wherein M is 0 in an appropriate non-reactive organic solvent or by reacting the appropriate haloauric acid hydrate which has been reduced by treatment with thiodiglycol with the appropriate compound of Formula (I) wherein M is 0, the following Formula (I) compounds wherein M is 1 and X is chloro or bromo are prepared; or by reacting the appropriate Formula (I) compound wherein M is 1 and X is chloro with sodium bromide in an appropriate organic solvent, such as aqueous ethanol or DMF, the following Formula (I) compounds wherein M is 1 and X is bromo are prepared; and by reacting the appropriate Formula (I) compound wherein M is 1 and X is chloro or bromo with sodium iodide in an appropriate organic solvent, such as acetone, the following Formula (I) compounds wherein M is 1 and X is iodo are prepared:

i) μ-[1,1-Bis(di-2-pyridylphosphino)methane]bis(chlorogold)
ii) μ-[1,3-Bis(di-2-pyridylphosphino)propane]bis(chlorogold)
iii) μ-[1,4-Bis(di-2-pyridylphosphino)butane])bis(chlorogold)
iv) μ-[1,5-Bis(di-2-pyridylphosphino)pentane]bis(chlorogold)
v) μ-[1,6-Bis(di-2-pyridylphosphino)hexane]bis(chlorogold)
vi) μ-[1,2-Bis(di-2-pyridylphosphino)ethane]bis(bromogold)
vii) μ-[1,2-Bis(di-2-pyridylphosphino)ethane]bis(iodogold)
viii) μ-[1,2-Bis(di-4-pyridylphosphino)methane]bis(chlorogold)
ix) μ-[1,2-Bis(di-4-pyridylphosphino)propane]bis(chlorogold)
x) μ-[1,2-Bis(di-4-pyridylphosphino)butane]bis(chlorogold)
xi) μ-[1,2-Bis(di-4-pyridylphosphino)pentane]bis(chlorogold)
xii) μ-[1,2-Bis(di-4-pyridylphosphino)hexane]bis(chlorogold)
xiii) μ-[1,2-Bis(di-4-pyridylphosphino)ethane]bis(bromogold)
xiv) μ-[1,2-Bis(di-4-pyridylphosphino)ethane]bis(iodogold).

b) Using the procedure outlined in Example 14, by reacting the appropriate Formula (I) compound, wherein R is 2-pyridyl, prepared as described above, with the appropriate Formula (I) compound wherein M is 0, prepared as described above, the following Formula (I) compounds wherein M is 1 are prepared:

i) Chlorobis[1,1-bis(di-2-pyridylphosphino)methane]gold
ii) Chlorobis[1,3-bis(di-2-pyridylphosphino)propane]gold
iii) Chlorobis[1,4-bis(di-2-pyridylphosphino)butane]gold
iv) Chlorobis[1,5-bis(di-2-pyridylphosphino)pentane]gold
v) Chlorobis[1,6-bis(di-2-pyridylphosphino)hexane]gold
vi) Iodobis[1,2-bis(di-2-pyridylphosphino)ethane]gold
vii) Bromobis[1,2-bis(di-2-pyridylphosphino)ethane]gold.

Example 16

Using the procedure of Example 12, by reacting the appropriate Formula (I) compound wherein X is chloro, prepared according to the procedure of Example 11, 13 or 15, with the appropriate thiosugar, the following Formula (I) compounds wherein X is thiosugar are prepared:

a) μ-[1,1-bis(di-2-pyridylphosphino)methane]bis-(1-thio-β-D-glucopyranosato-S)gold
b) μ-[1,3-bis(di-2-pyridylphosphino)propane]bis-(1-thio-β-D-glucopyranosato-S)gold
c) μ-[1,4-bis(di-2-pyridylphosphino)butane]bis-(1-thio-β-D-glucopyranosato-S)gold
d) μ-[1,5-bis(di-2-pyridylphosphino)pentane]bis-(1-thio-β-D-glucopyranosato-S)gold
e) μ-[1,6-bis(di-2-pyridylphosphino)hexane]bis-(1-thio-β-D-glucopyranosato-S)gold
f) μ-[1,2-bis(di-2-pyridylphosphino)ethane]bis-(1-thio-β-D-galactopyranosato-S)gold
g) μ-[1,2-bis(di-2-pyridylphosphino)ethane]bis-(1-thio-β-D-mannopyranosato-S)gold
h) μ-[1,2-bis(di-2-pyridylphosphino)ethane]bis-(1-thio-β-*D*-ribofuranosato-*S*)gold
i) μ-[1,1-bis(di-4-pyridylphosphino)methane]bis-(1-thio-β-D-glucopyranosato-S)gold
j) μ-[1,2-bis(di-4-pyridylphosphino)ethane]bis-(1-thio-β-D-glucopyranosato-S)gold
k) μ-[1,3-bis(di-4-pyridylphosphino)propane]bis-(1-thio-β-D-glucopyranosato-S)gold
l) μ-[1,4-bis(di-4-pyridylphosphino)butane]bis-(1-thio-β-D-glucopyranosato-S)gold
m) μ-[1,5-bis(di-4-pyridylphosphino)pentane]bis-(1-thio-β-D-glucopyranosato-S)gold
n) μ-[1,6-bis(di-4-pyridylphosphino)hexane]bis-(1-thio-β-D-glucopyranosato-S)gold
o) μ-[1,2-bis(di-4-pyridylphosphino)ethane]bis-(1-thio-β-D-galactopyranosato-S)gold
p) μ-[1,2-bis(di-4-pyridylphosphino)ethane]bis-(1-thio-β-D-mannopyranosato-S)gold
q) μ-[1,2-bis(di-4-pyridylphosphino)ethane]bis-(1-thio-*D*-ribofuranosato-*S*)gold.

Example 17

As a specific embodiment of a composition of this invention, an active ingredient, such as one part of the compound of Example 11, is dissolved in 5 parts of dimethylacetamide and 5 parts of polyethoxylated castor oil and then normal saline solution qs, and is administered parenterally in one dose of 5 $mg/m^2$ to a host animal afflicted with tumor cells sensitive to that compound.

**Claims for the Contracting States: BE CH DE FR GB IT LI LU NL SE**

1. A compound of the formula:

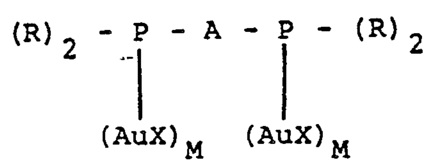

wherein:
R is the same and is 2-pyridyl, 4-pyridyl, 2-thienyl or 2-furyl;
X is the same and is halo or thiosugar;
M is 0 or 1, provided that when M is 1, R is 2-pyridyl or 4-pyridyl; and
A is a straight or branched alkanediyl chain of from one to six carbon atoms.

2. A compound of the formula:

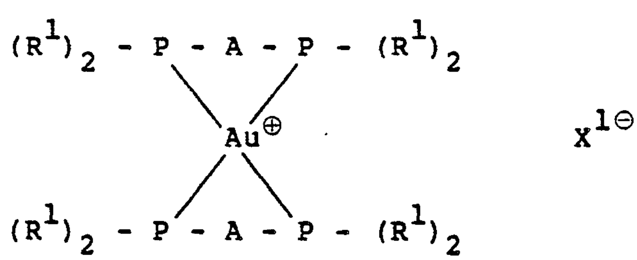

Formula II

wherein:
$R^1$ is the same and is 2-pyridyl;
A is the same and is a straight or branched alkanediyl chain from one to six carbon atoms; and

$X^1$ is the same and is halo.

3. A pharmaceutical composition which comprises a pharmaceutically acceptable carrier of diluent and a compound of the formula

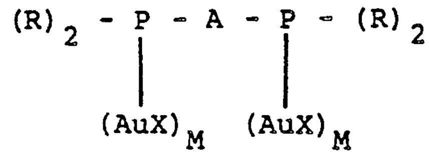

wherein:

R is the same and is 2-pyridyl, 4-pyridyl, 2-thienyl or 2-furyl;

X is the same and is halo or thiosugar;

M is 0 or 1, provided that when M is 1, R is 2-pyridyl or 4-pyridyl; and

A is a straight or branched alkanediyl chain of from one to six carbon atoms.

4. The composition of claim 3 wherein the composition is in a dosage unit form adapted for parenteral administration.

5. A pharmaceutical composition which comprises a pharmaceutically acceptable carrier or diluent, and a compound of the formula:

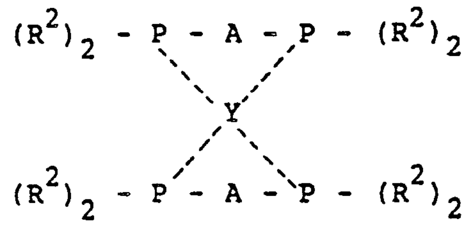

wherein:

$R^2$ is the same and is phenyl, ethyl or 2-pyridyl;

A is a straight or branched alkanediyl chain of from one to six carbon atoms;

Y is a link between the four P atoms of structure:

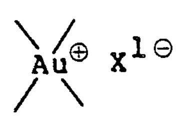

or

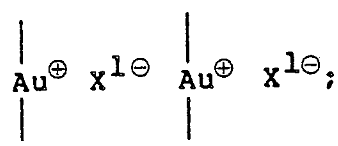

and $X^1$ is the same and is halo.

6. The composition of claim 5 wherein the composition is in a dosage form adapted for parenteral administration.

7. A pharmaceutical composition adapted for parenteral administration which comprises a pharmaceutically acceptable carrier or diluent and a compound of formula

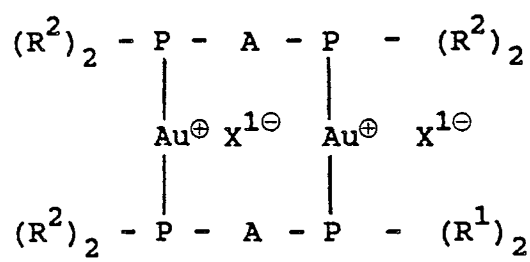

wherein:

$R^2$ is the same and is phenyl or ethyl;

A is a straight or branched alkanediyl chain of from 1 to 6 carbon atoms; and

$X^1$ is the same and is halo.

8. A process for preparing compound of the formula:

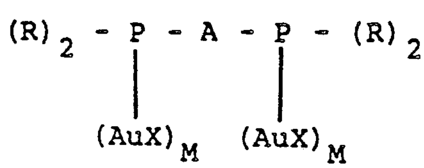

wherein:
R is the same and is 2-pyridyl, 4-pyridyl, 2-thienyl or 2-furyl;
X is the same and is halo or thiosugar;
M is 0 or 1; provided that when M is 1, R is 2-pyridyl or 4-pyridyl; and
A is a straight or branched alkanediyl chain of from one to six carbon atoms,
wherein such process comprises:

(a) reacting the appropriate heterocycle in an anhydrous ethyl ether with an organolithium reagent, such as n-butyl lithium, in a non-reactive organic solvent, with the appropriate compound of the formula:

$$Cl_2—P—A—P—Cl_2$$

wherein A is as defined above, to prepare the compounds of Formula (I) wherein M is 0;
(b) reacting the appropriate product of part (a), above, either directly with chloroauric acid tetrahydrate or a reduced form of the acid hydrate obtained by treatment with thiodiglycol in an appropriate non-reactive organic solvent to prepare the compounds of Formula (I) wherein M is 1 and X is chloro;
(c) reacting the appropriate product of part (b), above, with the appropriate sodium thiosugar to prepare the compounds of Formula (I) wherein M is 1 and X is thiosugar;
(d) (i) reacting the appropriate product of part (b), above, with sodium bromide in an appropriate organic solvent, such as aqueous ethanol or DMF; or
(ii) reacting the appropriate product of part (a), above, with the bromoauric acid hydrate which has been reduced by treatment with thiodiglycol; or
(iii) reacting the appropriate product of part (a), above, with bromoauric acid hydrate directly in an appropriate non-reactive organic solvent;
to prepare the compounds to Formula (I) wherein M is 1 and X is bromo; and
(e) reacting the appropriate product of part (b) or part (d) above, with sodium iodide in an appropriate organic solvent, such as acetone, to prepare the compounds of Formula (I) wherein M is 1 and X is iodo.

9. A process for preparing a compound of the formula:

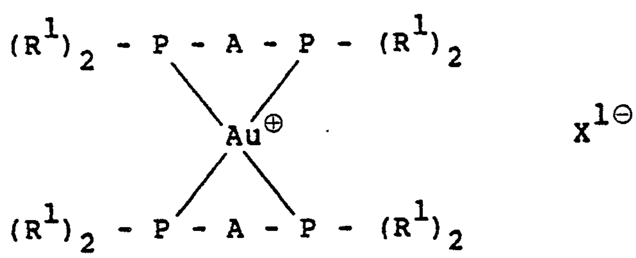

Formula II

wherein:
$R^1$ is the same and is 2-pyridyl;
A is the same and is a straight or branched alkanediyl chain from one to six carbon atoms; and
$X^1$ is the same and is halo;
wherein such process comprises reacting the appropriate compound of the formula:

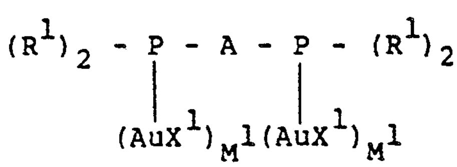

wherein:
$R^1$ is the same and is 2-pyridyl;
$X^1$ is the same and is halo;
$M^1$ is 0 or 1; and
A is a straight or branched alkanediyl chain of from one to six carbon atoms;
with the appropriate compound of the formula:

$$(R^1)_2—P—A—P—(R^1)_2$$

wherein $R^1$ and A are as defined above, in an appropriate non-reaactive organic solvent.

10. A compound according to claim 1 or claim 2 for use as a therapeutic agent.

11. A compound according to claim 1 or claim 2 for use in the inhibition of cell tumor growth.

12. A compound of formula

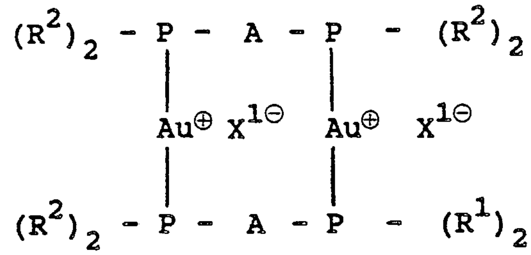

wherein:

$R^2$ is the same and is phenyl or ethyl;

A is a straight or branched alkanediyl chain of from 1 to 6 carbon atoms; and

$X^1$ is the same and is halo;

for use as a therapeutic agent.

**Claims for the Contracting State: AT**

1. A process for preparing a compound of the formula:

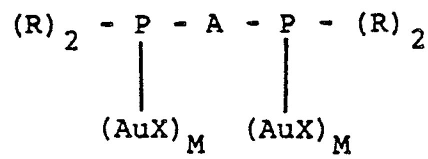

wherein:

R is the same and is 2-pyridyl, 4-pyridyl, 2-thienyl or 2-furyl;

X is the same and is halo or thiosugar;

M is 0 or 1; provided that when M is 1, R is 2-pyridyl or 4-pyridyl; and

A is a straight or branched alkanediyl chain of from one to six carbon atoms,

wherein such process comprises:

(a) reacting the appropriate heterocycle in an anhydrous ethyl ether with an organolithium reagent, such as n-butyl lithium, in a non-reactive organic solvent, with the appropriate compound of the formula:

$$Cl_2—P—A—P—Cl_2$$

wherein A is as defined above, to prepare the compounds of Formula (I) wherein M is 0;

(b) reacting the appropriate product of part (a), above, either directly with chloroauric acid tetrahydrate or a reduced form of the acid hydrate obtained by treatment with thiodiglycol in an appropriate non-reactive organic solvent to prepare the compounds of Formula (I) wherein M is 1 and X is chloro;

(c) reacting the appropriate product of part (b), above, with the appropriate sodium thiosugar to prepare the compounds of Formula (I) wherein M is 1 and X is thiosugar;

(d) (i) reacting the appropriate product of part (b), above, with sodium bromide in an appropriate organic solvent, such as aqueous ethanol or DMF; or

(ii) reacting the appropriate product of part (a), above, with the bromoauric acid hydrate which has been reduced by treatment with thiodiglycol; or

(iii) reacting the appropriate product of part (a), above, with bromoauric acid hydrate directly in an appropriate non-reactive organic solvent;

to prepare the compounds to Formula (I) wherein M is 1 and X is bromo; and

(e) reacting the appropriate product of part (b) or part (d), above, with sodium iodide in an appropriate organic solvent, such as acetone, to prepare the compounds of Formula (I) wherein M is 1 and X is iodo.

2. A process for preparing a compound of the Formula:

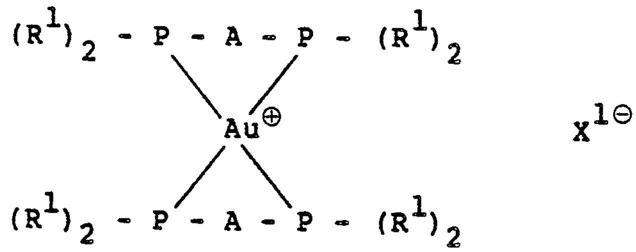

Formula II

wherein:

$R^1$ is the same and is 2-pyridyl;

A is the same and is a straight or branched alkanediyl chain from one to six carbon atoms; and

$X^1$ is the same and is halo;

wherein such process comprises reacting the appropriate compound of the formula:

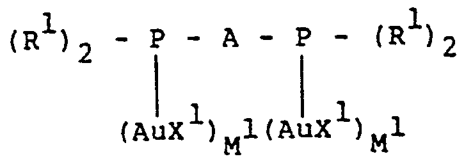

wherein:

$R^1$ is the same and is 2-pyridyl;

$X^1$ is the same and is halo;

$M^1$ is 0 or 1; and

A is a straight or branched alkanediyl chain of from one to six carbon atoms;

with the appropriate compound of the formula:

$$(R^1)_2—P—A—P—(R^1)_2$$

wherein $R^1$ and A are as defined above in an appropriate non-reactive organic solvent.

3. A process for preparing a pharmaceutical composition which comprises admixing a pharmaceutically acceptable carrier or diluent and a compound of the formula:

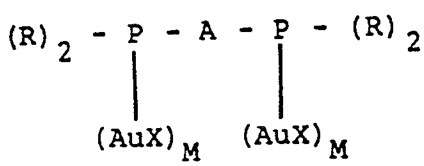

wherein:

R is the same and is 2-pyridyl, 4-pyridyl, 2-thienyl or 2-furyl;

X is the same and is halo or thiosugar;

M is 0 or 1, provided that when M is 1, R is 2-pyridyl or 4-pyridyl; and

A is a straight or branched alkanediyl chain of from one to six carbon atoms.

4. The process of claim 3 wherein the composition is in a dosage unit form adapted for parenteral administration.

5. A process for preparing a pharmaceutical composition which comprises admixing a pharmaceutically acceptable carrier or diluent and a compound of the formula:

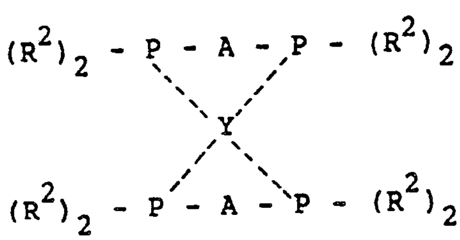

(III)

wherein:

$R^2$ is the same and is 2-pyridyl;

A is a straight or branched alkanediyl chain of from 1 to 6 carbon atoms;

Y is a link between the four P atoms of structure:

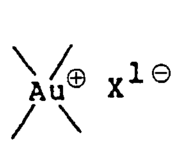 or 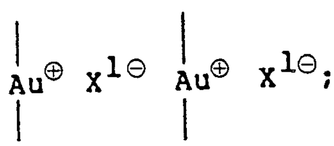;

and $X^1$ is the same and is halo.

6. The process of claim 5 wherein the composition is in a dosage unit form adapted for parenteral administration.

7. A process for preparing a parenteral pharmaceutical composition which comprises admixing a pharmaceutically acceptable carrier or diluent and a compound of formula

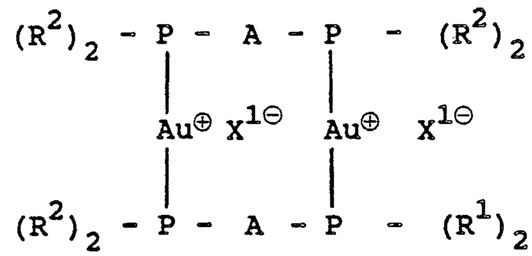

wherein:

$R^2$ is the same and is phenyl or ethyl;

A is a straight or branched alkanediyl chain of from 1 to 6 carbon atoms; and

$X^1$ is the same and is halo.

**Patentansprüche für die Vertragsstaaten: BE CH DE FR GB IT LI LU NL SE**

1. Eine Verbindung der Formel

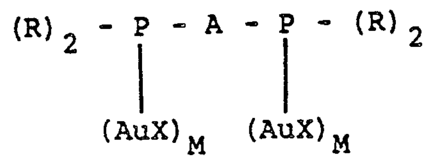

in der:

die Reste R die gleiche Bedeutung haben und 2-Pyridyl-, 4-Pyridyl-, 2-Thienyl- oder 2-Furylgruppen sind;

die Reste X die gleiche Bedeutung haben und Halogen- oder Thiozuckerreste sind;

M 0 oder 1 ist, mit der Maßgabe, daß R eine 2-Pyridyl- oder 4-Pyridylgruppe bedeutet, wenn M 1 ist und A eine unverzweigte oder verzweigte Alkandiylkette mit 1 bis 6 Kohlenstoffatomen bedeutet.

2. Eine Verbindung der Formel

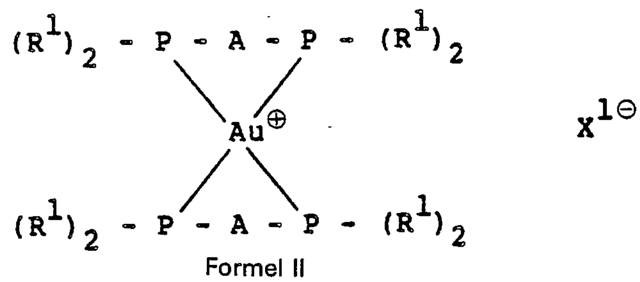

Formel II

in der:

die Reste $R^1$ gleich sind und eine 2-Pyridylgruppe bedeuten,

die Reste A gleich sind und eine unverzweigte oder verzweigte Alkandiylkette mit 1 bis 6 Kohlenstoffatomen bedeuten; und

die Reste $X^1$ gleich sind und Halogenatome bedeuten.

3. Arzneimittel, umfassend einen pharmazeutisch verträglichen Träger oder ein Verdünnungsmittel und eine Verbindung der Formel

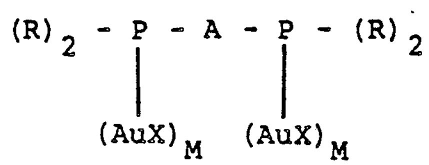

in der:

die Reste R die gleiche Bedeutung haben und 2-Pyridyl-, 4-Pyridyl-, 2-Thienyl- oder 2-Furylgruppen sind; die Reste X die gleiche Bedeutung haben und Halogen- oder Thiozuckerreste sind;

M 0 oder 1 ist, mit der Maßgabe, daß R eine 2-Pyridyl- oder 4-Pyridylgruppe bedeutet, wenn M 1 ist und A eine unverzweigte oder verzweigte Alkandiylkette mit 1 bis 6 Kohlenstoffatomen bedeutet.

4. Mittel nach Anspruch 3, wobei das Mittel in einer Dosiseinheitsform zur parenteralen Verabreichung vorliegt.

5. Arznemittel, umfassend einen pharmazeutisch verträglichen Träger oder ein Verdünngsmittel, une eine Verbindung der Formel

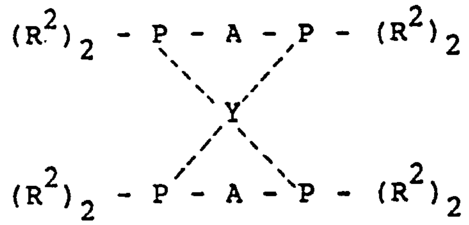

in der:

die Reste $R^2$ gleich sind und eine 2-Pyridylgruppe bedeuten;
A eine unverzweigte oder verzweigte Alkandiylkette mit 1 bis 6 Kohlenstoffatomen darstellt;
Y eine Verbindung zwischen den vier P-Atomen mit der Struktur

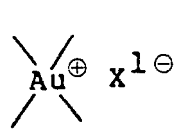 oder 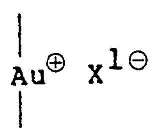 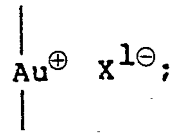

bedeutet; und

die Reste $X^1$ gleich sind und Halogenatome bedeuten.

6. Mittel nach Anspruch 5, wobei das Mittel in einer Dosisform zur parenteralen Verabreichung vorliegt.

7. Arnzeimittel zur parenteralen Verabreichung, welches einen pharmazeutisch verträglichen Träger oder ein Verdünngsmittel und eine Verbindung der Formel

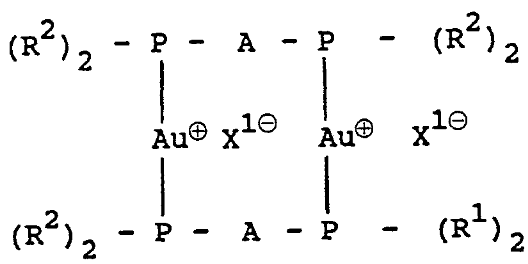

umfaßt, in der:

die Reste $R^2$ gleich sind und Phenyl- oder Äthylgruppen bedeuten;
A eine unverzweigte oder verzweigte Alkandiylkette mit 1 bis 6 Kohlenstoffatom darstellt; und
die Reste $X^1$ gleich sind und Halogenatome bedeuten.

8. Verfahren zur Herstellung einer Verbindung der Formel

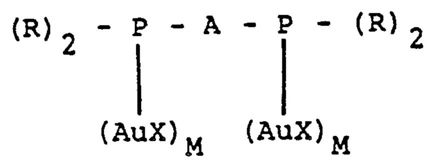

in der:

die Reste R die gleiche Bedeutung haben und 2-Pyridyl-, 4-Pyridyl-, 2-Thienyl- oder 2-Furylgruppen sind;
die Reste X die gleiche Bedeutung haben und Halogen- oder Thiozuckerreste sind;
M 0 oder 1 ist, mit der Maßgabe, daß R eine 2-Pyridyl- oder 4-Pyridylgruppe bedeutet, wenn M 1 ist und
A eine unverzweigte oder verzweigte Alkandiylkette mit 1 bis 6 Kohlenstoffatomen bedeutet,

wobei das Verfahren umfaßt:

(a) Umsetzung des entsprechenden Heterocyclus in einem wasserfreien Äthyläther mit einem Organolithium-Reagens, wie n-Butyllithium, in einem nicht-reaktiven organischen Lösungsmittel mit der entsprechenden Verbindung der Formel:

$$Cl_2—P—A—P—Cl_2$$

in der A wie vorstehend definiert ist, zur Herstellung der Verbindungen der Formel (I), in denen M 0 ist,

(b) Umsetzung des entsprechenden Produktes vom vorstehenden Teil (a), entweder direkt mit Chlorgoldsäure-Tetrahydrat oder mit einer reduzierten Form des Säurehydrates, die durch Behandlung mit Thiodiglykol in einem entsprechenden nicht-reaktiven organischen Lösungsmittel erhalten wird, zur Herstellung der Verbindung der Formel (I), in denen M 1 ist und X ein Chloratom bedeutet;

(c) Umsetzung des entsprechenden Produktes des vorstehenden Teils (b) mit dem entsprechenden Natriumthiozucker zur Herstellung der Verbindungen der Formel (I), in denen M 1 ist und X einen Thiozuckerrest bedeutet;

(d) (i) Umsetzung des entsprechenden Produktes des vorstehenden Teils (b) mit Natriumbromid in einem geeigneten organischen Lösungsmittel, wie wäßrigem Äthanol oder DMF; oder

(ii) des entsprechenden Produktes des vorstehenden Teils (a) mit dem Bromgoldsäurehydrat, das durch Behandlung mit Thiodiglykol reduziert worden ist; oder

(iii) Umsetzung des entsprechenden Produktes des vorstehenden Teils (a) mit Bromgoldsäurehydrat direkt in einem entsprechenden nicht-reaktiven organischen Lösungsmittel;

zur Herstellung der Verbindungen der Formel (I), in denen M 1 ist und X ein Bromatom bedeutet; und

(e) Umsetzung des entsprechenden Produktes des vorstehenden Teils (b) oder Teils (d) mit Natriumjodid in einem entsprechenden organischen Lösungsmittel, wie Aceton, zur Herstellung der Verbindungen der Formel (I), in denen M 1 ist und X ein Jodatom bedeutet.

9. Verfahren zur Herstellung einer Verbindung der Formel

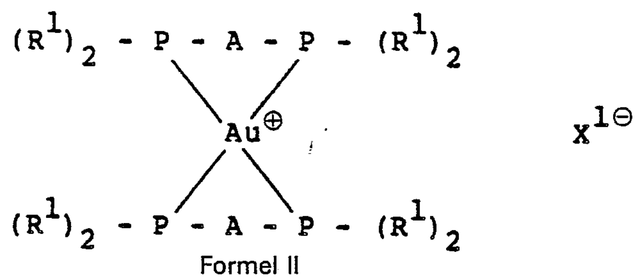

Formel II

in der;

die Reste $R^1$ gleich sind und eine 2-Pyridylgruppe bedeuten,

die Reste A gleich sind und eine unverzweigte oder verzweigte Alkandiylkette mit 1 bis 6 Kohlenstoffatomen bedeuten; und

die Reste $X^1$ gleich sind und Halogenatome bedeuten,

wobei das Verfahren umfaßt die Umsetzung der entsprechenden Verbindung der Formel

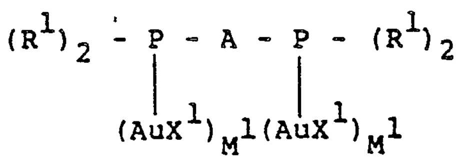

in der:

die Reste $R^1$ gleich sind und eine 2-Pyridylgruppe bedeuten;

die Reste $X^1$ gleich sind und Halogenatome bedeuten,

$M^1$ 0 oder 1 ist; und

A eine unverzweigte oder verzweigte Alkandiylkette mit 1 bis 6 Kohlenstoffatomen darstellt;

mit der entsprechenden Verbindung der Formel

$$(R^1)_2—P—A—P—(R^1)_2$$

in der $R^1$ und A wie vorstehend definiert sind, in einem entsprechenden nicht-reaktiven organischen Lösungsmittel.

10. Verbindung nach Anspruch 1 oder 2 zur Verwendung als therapeutischer Wirkstoff.

11. Verbindung nach Anspruch 1 oder 2 zur Verwendung bei der Inhibierung des Zelltumor-Wachstums.

12. Eine Verbindung der Formel

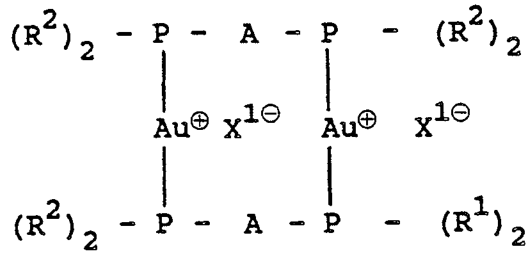

in der;

die Reste $R^2$ gleich sind und Phenyl- oder Äthylgruppen bedeuten;

A eine unverzweigte oder verzweigte Alkandiylkette mit 1 bis 6 Kohlenstoffatomen darstellt; und die Reste $X^1$ gleich sind und Halogenatome bedeuten, zur Verwendung als therapeutischer Wirkstoff.

**Patentansprüche für den Vertragsstaat: AT**

1. Verfahren zur Herstellung einer Verbindung der Formel

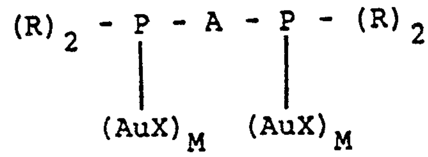

in der:
die Reste R die gleiche Bedeutung haben und 2-Pyridyl-, 4-Pyridyl-, 2-Thienyl- oder 2-Furylgruppen sind;
die Reste X die gleiche Bedeutung haben und Halogen- oder Thiozuckerreste sind;
M 0 oder 1 ist, mit der Maßgabe, daß R eine 2-Pyridyl- oder 4-Pyridylgruppe bedeutet, wenn M 1 ist und
A eine unverzweigte oder verzweigte Alkandiylkette mit 1 bis 6 Kohlenstoffatomen bedeutet,
wobei das Verfahren umfaßt:
(a) Umsetzung des entsprechenden Heterocyclus in einem wasserfreien Äthyläther mit einem Organolithium-Reagens, wie n-Butyllithium, in einem nicht-reaktiven organischen Lösungsmittel mit der entsprechenden Verbindung der Formel:

$$Cl_2—P—A—P—Cl_2$$

in der A wie vorstehend definiert ist, zur Herstellung der Verbindungen der Formel (I), in denen M 0 ist,
(b) Umsetzung des entsprechenden Produktes vom vorstehenden Teil (a), entweder direkt mit Chlorgoldsäure-Tetrahydrat oder mit einer reduzierten Form des Säurehydrates, die durch Behandlung mit Thiodiglykol in einem entsprechenden nicht-reaktiven organischen Lösungsmittel erhalten wird, zur Herstellung der Verbindung der Formel (I), in denen M 1 ist und X ein Chloratom bedeutet;
(c) Umsetzung des entsprechenden Produktes des vorstehenden Teils (b) mit dem entsprechenden Natrium-thiozucker zur Herstellung der Verbindungen der Formel (I), in denen M 1 ist und X einen Thiozuckerrest bedeutet;
(d) (i) Umsetzung des entsprechenden Produktes des vorstehenden Teils (b) mit Natriumbromid in einem geeigneten organischen Lösungsmittel, wie wäßrigem Äthanol oder DMF; oder
(ii) des entsprechenden Produktes des vorstehenden Teils (a) mit dem Bromgoldsäurehydrat, das durch Behandlung mit Thiodiglykol reduziert worden ist; oder
(iii) Umsetzung des entsprechenden Produktes des vorstehenden Teils (a) mit Bromgoldsäurehydrat direkt in einem entsprechenden nicht-reaktiven organischen Lösungsmittel;
zur Herstellung der Verbindungen der Formel (I), in denen M 1 ist und X ein Bromatom bedeutet; und
(e) Umsetzung des entsprechenden Produktes des vorstehenden Teils (b) oder Teils (d) mit Natriumjodid in einem entsprechenden organischen Lösungsmittel, wie Aceton, zur Herstellung der Verbindungen der Formel (I), in denen M 1 ist und X ein Jodatom bedeutet.

2. Verfahren zur Herstellung einer Verbindung der Formel

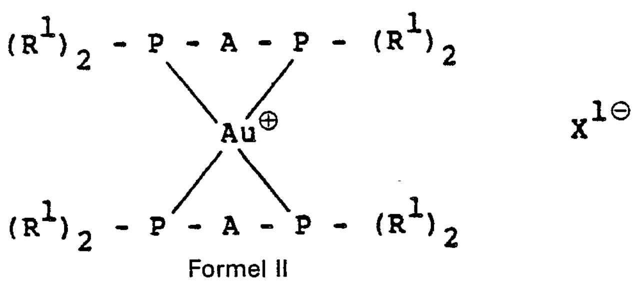

Formel II

in der;
die Reste $R^1$ gleich sind und eine 2-Pyridylgruppe bedeuten,
die Reste A gleich sind und eine unverzweigte oder verzweigte Alkandiylkette mit 1 bis 6 Kohlenstoffatomen bedeuten; und
die Reste $X^1$ gleich sind und Halogenatome bedeuten,
wobei das Verfahren umfaßt die Umsetzung der entsprechenden Verbindung der Formel

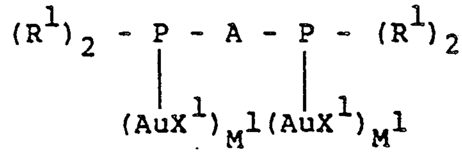

in der:
die Reste $R^1$ gleich sind und eine 2-Pyridylgruppe bedeuten;
die Reste $X^1$ gleich sind und Halogenatome bedeuten,
$M^1$ 0 oder 1 ist; und
A eine unverzweigte oder verzweigte Alkandiylkette mit 1 bis 6 Kohlenstoffatomen darstellt;
mit der entsprechenden Verbindung der Formel

$$(R^1)_2—P—A—P—(R^1)_2$$

in der $R^1$ und A wie vorstehend definiert sind, in einem entsprechenden nicht-reaktiven organischen Lösungsmittel.

3. Verfahren zur Herstellung eines Arzneimittels, das Mischen eines pharmazeutisch verträglichen Trägers oder Verdünnungsmittels und einer Verbindung der Formel

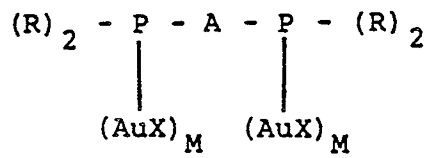

umfaßt, in der
die Reste R die gleiche Bedeutung haben und 2-Pyridyl-, 4-Pyridyl-, 2-Thienyl- oder 2-Furylgruppen sind;
die Reste X die gleiche Bedeutung haben und Halogen- oder Thiozuckerreste sind;
M 0 oder 1 ist, mit der Maßgabe, daß R eine 2-Pyridyl- oder 4-Pyridylgruppe bedeutet, wenn M 1 ist und
A eine unverzweigte oder verzweigte Alkandiylkette mit 1 bis 6 Kohlenstoffatomen bedeutet.

4. Verfahren nach Anspruch 3, wobei das Mittel in einer Einheitsdosisform zur parenteralen Verabreichung vorliegt.

5. Verfahren zur Herstellung eines Arzneimittels, das Vermischen eines pharmazeutisch verträglichen Trägers oder Verdünnungsmittels und einer Verbindung der Formel

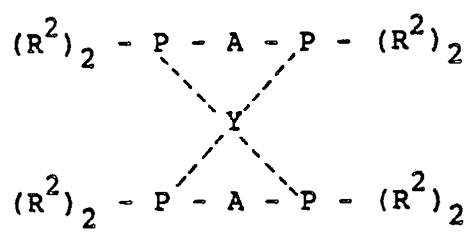

(III)

umfaßt, wobei:
die Reste $R^2$ gleich sind und eine 2-Pyridylgruppe bedeuten;
A eine unverzweigte oder verzweigte Alkandiylkette mit 1 bis 6 Kohlenstoffatomen darstellt;
Y eine Verbindung zwischen den vier P-Atomen mit der Struktur

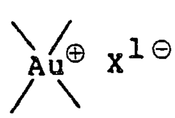

oder

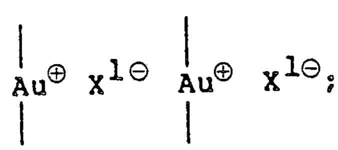

bedeutet; und
die Reste $X^1$ gleich sind und Halogenatome bedeuten.

6. Verfahren nach Anspruch 5, wobei das Mittel in einer Einheitsdosisform zur partenteralen Verabreichung vorliegt.

7. Verfahren zur Herstellung eines parenteralen Arzneimittels, das Mischen eines pharmazeutisch verträglichen Trägers oder eines Verdünnungsmittels und einer Verbindung der Formel

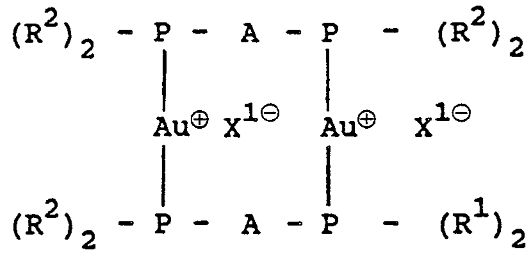

umfaßt, in der

die Reste $R^2$ gleich sind und Phenyl- oder Äthylgruppen bedeuten;

A eine unverzweigte oder verzweigte Alkandiylkette mit 1 bis 6 Kohlenstoffatomen darstellt; und

die Reste $X^1$ gleich sind und Halogenatome bedeuten.

**Revendications pour les Etats contractants: BE CH DE FR GB IT LI LU NL SE**

1. Composé de formule:

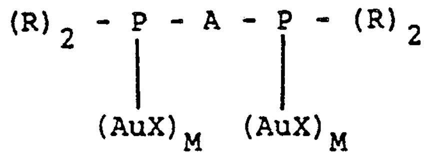

dans laquelle:

R est le même et est 2-pyridyle, 4-pyridyle, 2-thiényle ou 2-furyle;

X est le même et est halo ou un sucre avec groupement thio;

M est 0 ou 1; à condition que, lorsque M est 1, R soit 2-pyridyle ou 4-pyridyle; et

A est une chaîne alcanediyle droite ou ramifiée de un à six atomes de carbone.

2. Composé de formule:

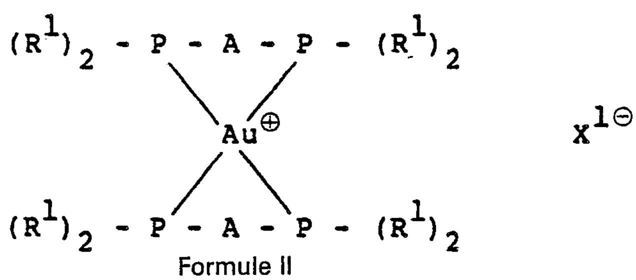

Formule II

dans laquelle

$R^1$ est le même et est 2-pyridyle;

A est le même et est une chaîne aclanediyle droite ou ramifiée de un à six atomes de carbone; et

$X^1$ est le même et est halo.

3. Composition pharmaceutique qui comprend un support ou diluant pharmaceutiquement acceptable et un composé de formule

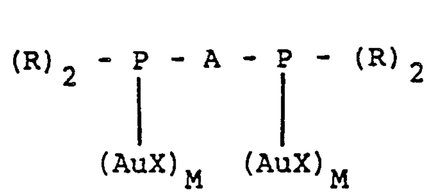

dans laquelle

R est le même et est 2-pyridyle, 4-pyridyle, 2-thiényle our 2-furyle;

X est le même et est halo ou un sucre avec groupement thio;

M est 0 ou 1; à condition que, lorsque M est 1, R soit 2-pyridyle ou 4-pyridyle; et

A est une chaîne alcanediyle droite ou ramifiée de un à six atomes de carbone.

4. Composition de la revendication 3 dans laquelle la composition est sous la forme d'une dose unitaire adaptée à l'administration parentérale.

5. Composition pharmaceutique qui comprend un support ou diluant pharmaceutique acceptable et un composé de formule:

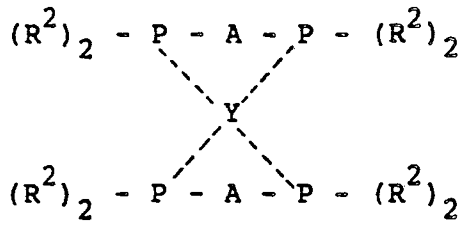

dans laquelle:

$R^2$ est le même et est 2-pyridyle;

A est un chaîne alcanediyle droite ou ramifiée de un à six atomes de carbone;

Y est un lien entre les quatre atomes P de structure:

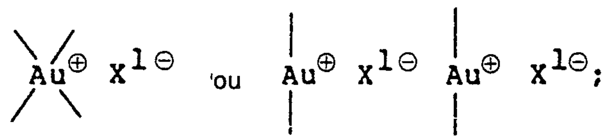

et $X^1$ est le même et est halo.

6. Composition de la revendication 5 dans laquelle la composition est sous une forme de dosage adaptée à l'administration parentérale.

7. Composition pharmaceutique adaptée à l'administration parentérale qui comprend un support ou diluant pharmaceutiquement acceptable et un composé de formule:

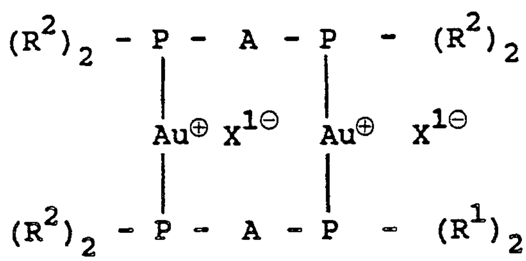

dans laquelle

$R^2$ est le même et est phényle ou éthyle

A est un chaîne alcanediyle droite ou ramifiée de un à six atomes de carbone; et

$X^1$ est le même et est halo.

8. Procédé pour préparer un composé de formule:

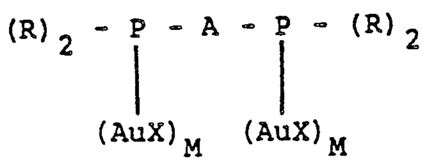

dans laquelle;

R est le même et est 2-pyridyle, 4-pyridyle, 2-thiényle ou 2-furyle;

X est le même et est halo ou un sucre avec groupement thio;

M est 0 ou 1; à condition que, lorsque M est 1, R soit 2-pyridyle ou 4-pyridyle; et

A est une chaîne alcanediyle droite ou ramifiée de un à six atomes de carbone

où ledit procédé comprend:

(a) la réaction de l'hétérocycle approprié dans un éther éthylique anhydre avec un réactif organolithium tel que le n-butyl lithium dans un solvant organique non réactif avec le composé appropriaté de formule:

$$Cl_2—P—A—P—Cl_2$$

dans laquelle A est tel que défini plus haut, pour préparer les composés de Formule (I) dans laquelle M est O;

(b) la réaction du produit approprié de la partie (a) ci-dessus, soit directement avec le tétrahydrate d'acide chloroaurique soit avec une forme réduite de l'hydrate de l'acide obtenue par traitement avec du thiodiglycol dans un solvant organique non-réactif approprié pour préparer les composés de Formule (I) où M est 1 est X est chloro.

(c) la réaction du produit approprié de la partie (b) ci-dessus avec le dérivé sodique de sucre avec un groupement thio approprié pour préparer les composés de Formule (I) où M est 1 et X est un sucre avec groupement thio;

(d) (i) la réaction du produit approprié de la partie (b) ci-dessus avec du bromure de sodium dans un solvant organique approprié tel que l'éthanol aqueux ou le DMF; ou

(ii) la réaction du produit approprié de la partie (a) ci-dessus avec l'hydrate de l'acide bromoaurique qui a été réduit par traitement avec du thiodigylcol; ou

(iii) la réaction du produit approprié de la partie (a) ci-dessus avec l'hydrate de l'acide bromoaurique directement dans un solvant organique non-réactif approprié;

pour préparer les composés de formule (I) dans laquelle M est 1 est X bromo, et

(e) la réaction du produit approprié de la partie (b) ou (d) ci-dessus avec de l'iodure de sodium dans un solvant organique approprié tel que l'acétone pour préparer les composés de Formule (I) dans laquelle M est 1 et X est iodo.

9. Procédé pour préparer un composé de formule:

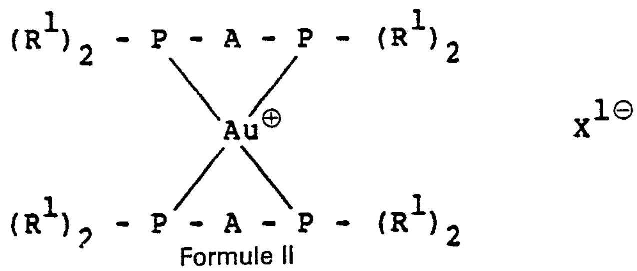

Formule II

dans laquelle;

$R^1$ est le même et est 2-pyridyle;

A est le même et est une chaîne alcanediyle droite ou ramifiée de un à six atomes de carbone;

$X^1$ est le même est halo;

lequel procédé comprend la réaction du composé approprié de formule:

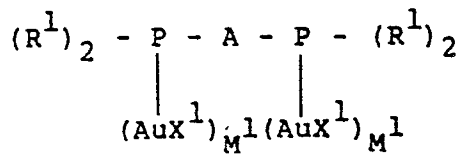

dans laquelle:

$R^1$ est le même et est 2-pyridyle;

$X^1$ est le même et est halo;

$M^1$ est 0 ou 1; et

A est une chaîne alcanediyle droite ou ramifiée de un à six atomes de carbone;

avec le composé approprié de formule:

$$(R^1)_2—P—A—P—(R^1)_2$$

dans laquelle $R^1$ est A sont tels que définis plus haut, dans un solvant organique non-réactif approprié.

10. Composé suivant la revendication 1 ou la revendication 2 pour usage comme agent thérapeutique.

11. Composé suivant la revendication 1 ou la revendication 2 pour usage dans l'inhibition de la croissance de cellules tumorales.

12. Composé de formule:

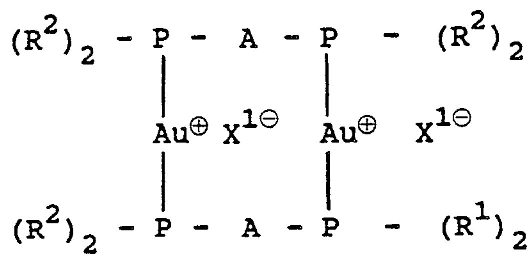

dans laquelle:

$R^2$ est le même et est phényle ou éthyle;

A est une chaîne alcanediyle droite ou ramifiée de un à six atomes de carbone; et

$X^1$ est le même et est halo;

pour usage comme agent thérapeutique.

**Revendications pour l'Etat contractant: AT**

1. Procédé pour préparer un composé de formule:

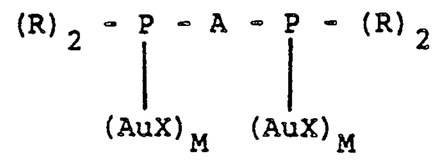

dans laquelle:

R est le même et est 2-pyridyle, 4-pyridyle, 2-thiényle ou 2-furyle;

X est le même et est halo ou un sucre avec groupement thio;

M est 0 ou 1 à condition que, lorsque M est 1, R soit 2-pyridyle ou 4-pyridyle; et

A est une chaîne alcanediyle droite ou ramifiée de un à six atomes de carbone

où ledit procédé comprend:

(a) la réaction de l'hétérocycle approprié dans un éther éthylique anhydre avec un réactif organolithium tel que le n-butyl lithium dans un solvant organique non réactif avec le composé appropriaté de formule:

$$Cl_2—P—A—P—Cl_2$$

dans laquelle A est tel que défini plus haut, pour préparer les composés de Formule (I) dans laquelle M est O;

(b) la réaction du produit approprié de la partie (a) ci-dessus, soit directement avec le tétrahydrate d'acide chloroaurique soit avec une forme réduite de l'hydrate de l'acide obtenue par traitement avec du thiodiglycol dans un solvant organique non-réactif approprié pour préparer les composés de Formule (I) où M est 1 et X est chloro.

(c) la réaction du produit approprié de la partie (b) ci-dessus avec le dérivé sodique de sucre avec un groupement thio·approprié pour préparer les composés de Formule (I) où M est 1 et X est un sucre avec groupement thio;

(d) (i) la réaction du produit approprié de la partie (b) ci-dessus avec du bromure de sodium dans un solvant organique approprié tel que l'éthanol aqueux ou le DMF; ou

(ii) la réaction du produit approprié de la partie (a) ci-dessus avec l'hydrate de l'acide bromoaurique qui a été réduit par traitement avec du thiodigylcol; ou

(iii) la réaction du produit approprié de la partie (a) ci-dessus avec l'hydrate de l'acide bromoaurique directement dans un solvant organique non-réactif approprié;

pour préparer les composés de formule (I) dans laquelle M est 1 est X bromo, et

(e) la réaction du produit approprié de la partie (b) ou (d) ci-dessus avec de l'iodure de sodium dans un solvant organique approprié tel que l'acétone pour préparer les composés de Formule (I) dans laquelle M est 1 et X est iodo.

2. Procédé pour préparer un composé de formule:

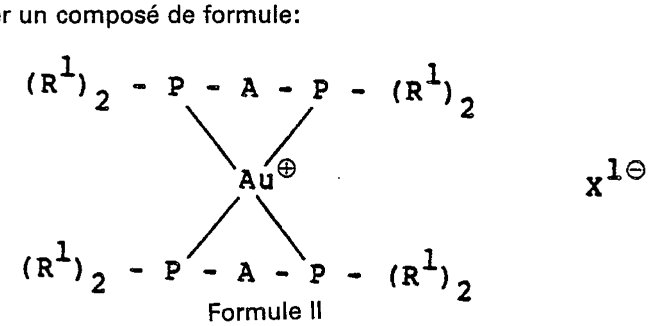

Formule II

dans laquelle:

$R^1$ est le même et est 2-pyridyle;

A est le même et est une chaîne alcanediyle droite ou ramifiée de un à six atomes de carbone;

$X^1$ est le même est halo;

où ledit procédé comprend la réaction du composé approprié de formule:

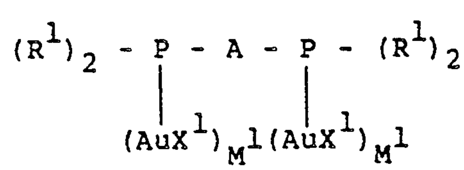

dans laquelle:

$R^1$ est le même et est 2-pyridyle;

$X^1$ est le même et est halo;

$M^1$ est 0 ou 1; et

A est une chaîne alcanediyle droite ou ramifiée de un à six atomes de carbone;

avec le composé approprié de formule:

$$(R^1)_2—P—A—P—(R^1)_2$$

dans laquelle $R^1$ est A sont tels que définis plus haut, dans un solvant organique non-réactif approprié.

3. Procédé pour préparer une composition pharmaceutique qui comprend le mélange d'un support ou diluant pharmaceutiquement acceptable et d'un composé de formule

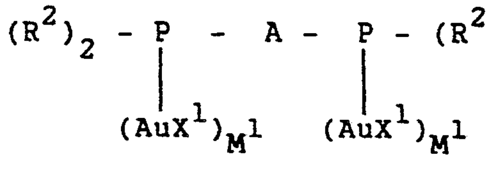

dans laquelle:

R est le même et est 2-pyridyle, 4-pyridyle, 2-thiényle ou 2-furyle;

X est le même et est halo ou un sucre avec groupement thio;

M est 0 ou 1; à condition que, lorsque M est 1, R soit 2-pyridyle ou 4-pyridyle; et

A est une chaîne alcanediyle droite ou ramifiée de un à six atomes de carbone.

4. Procédé de la revendication 3 dans laquelle la composition est sous une forme de dosage unitaire adaptée à l'administration parentérale.

5. Procédé préparer une composition pharmaceutique qui comprend le mélange d'un support ou diluant pharmaceutiquement acceptable et d'un composé de formule:

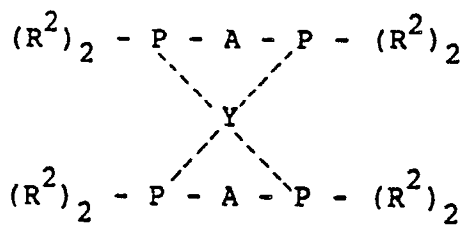

dans laquelle:

$R^2$ est le même et est 2-pyridyle;

A est une chaîne alcanediyle droite ou ramifiée de un à six atomes de carbone;

Y est un lien entre les quatre atomes P de structure:

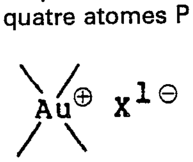

ou

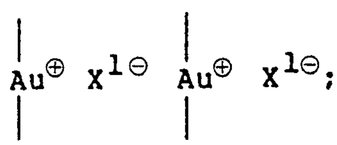

et $X^1$ est le même et est halo.

6. Procédé de la revendication 5 dans laquelle la composition est sous la forme d'une unité de dosage adaptée à l'administration parentérale.

7. Procédé pour préparer une composition pharmaceutique parentérale qui comprend le mélange d'un support ou diluant pharmaceutiquement acceptable et d'un composé de formule:

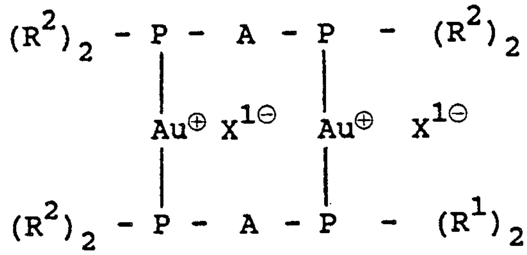

dans laquelle

$R^2$ est le même et est phényle ou éthyle

A est un chaîne alcanediyle droite ou ramifiée de un à six atomes de carbone; et

$X^1$ est le même et est halo.